**Europäisches Patentamt**

**European Patent Office**

**Office européen des brevets**

(19)

(11) Veröffentlichungsnummer: **0 340 540 B1**

(12) # EUROPÄISCHE PATENTSCHRIFT

(45) Veröffentlichungstag der Patentschrift:
**30.10.91 Patentblatt 91/44**

(51) Int. Cl.⁵: **A61K 33/14, A61K 31/61,** **A61K 31/615, // (A61K33/14, 31:60, 31:195)**

(21) Anmeldenummer: **89107059.1**

(22) Anmeldetag: **19.04.89**

(54) Mittel zur Prophylaxe des Herzinfarkts und zur Verhütung des Reinfarkts.

(30) Priorität: **02.05.88 DE 3814856**

(43) Veröffentlichungstag der Anmeldung:
**08.11.89 Patentblatt 89/45**

(45) Bekanntmachung des Hinweises auf die Patenterteilung:
**30.10.91 Patentblatt 91/44**

(84) Benannte Vertragsstaaten:
**AT BE CH DE ES FR GB GR IT LI LU NL SE**

(56) Entgegenhaltungen:
**EP-A- 0 108 937**
**FR-A- 2 187 763**
**CHEMICAL ABSTRACTS, Band 88, Nr. 6, 6. Februar 1978, Seite 252, Zusammenfassung Nr. 41680g, Columbus, Ohio, US; & JP-A-77 70 011 (VERLA-PHARM. ARZNEIMITTELFABRIK APOTHEKER H.J.V. EHRLICH) 10-06-1977**

(73) Patentinhaber: **Verla-Pharm Arzneimittelfabrik Apotheker H.J. von Ehrlich GmbH & Co. KG Hauptstrasse 98 W-8132 Tutzing (DE)**

(72) Erfinder: **Helbig, Joachim, Dr. Traubinger Strasse 47 W-8132 Tutzing (DE)**
Erfinder: **Classen, Hans Georg, Prof.Dr. Fruwirthstrasse 30 W-7000 Stuttgart 70 (DE)**

(74) Vertreter: **Patentanwälte Deufel, Hertel, Lewald Isartorplatz 6 Postfach 26 02 47 W-8000 München 26 (DE)**

EP 0 340 540 B1

## Beschreibung

Die Erfindung betrifft ein Mittel zur Prophylaxe des Herzinfarkts und zur Verhütung des Reinfarkts.

Es ist bekannt, daß Magnesium$^{2+}$-Ionen ein breites cardiovaskuläres Wirkungsspektrum, das in erster Linie auf ihren fundamentalen Ca(II)-antagonistischen Eigenschaften beruht, besitzen. Diese Wirkung ist besonders ausgeprägt bei Magnesiumaminodicarbonsäurehalogeniden, wie Magnesium-L-Aspartat-Hydrochlorid und Magnesium-L-Glutamat-Hydrochlorid, die eine hohe Resorptionsquote zeigen. Zusätzlich zum Herzzellschutz durch Calciumantagonismus besitzen diese Verbindungen noch einen spasmolytischen Effekt auf die Koronararterien sowie eine streßabschirmende Wirkung.

Obwohl aufgrund des calciumantagonistischen Effekts immer wieder postuliert wird, daß Magnesiumverbindungen auch eine Thrombozytenaggregationshemmende Wirkung hätten, zeigen klinische Untersuchungen, daß dies nicht der Fall ist.

Nachdem die Magnesiumverbindungen keine Thrombozytenaggregation-hemmende Wirkung besitzen, jedoch aufgrund der immer mehr zunehmenden Herzinfarkte und Reinfarkte ein erheblicher Bedarf an einem auf möglichst breiter Wirkungsbasis beruhenden Mittel zur Infarktprophylaxe und Reinfarktprophylaxe besteht, hatten sich die Erfinder die Aufgabe gestellt, ein Mittel zur Infarktprophylaxe und Reinfarktprophylaxe zu schaffen, das alle möglichen biochemischen Ursachen des Herzinfarkts bzw. Reinfarkts bekämpft.

Diese Aufgabe wurde durch das erfindungsgemäße Mittel zur Prophylaxe des Herzinfarkts und zur Verhütung eines Reinfarkts gelöst, welches dadurch gekennzeichnet ist, daß es aus einer Kombination aus Magnesiumdicarbonsäurehalogeniden, basischen Magnesiumaminodicarbonsäuresalzen und Magnesiumhalogeniden einzeln oder in Kombination einerseits und Acetylsalicylsäure andererseits sowie gegebenenfalls üblichen pharmazeutischen Trägern, Verdünnungsmittel, und/oder Hilfsstoffen besteht.

Durch die erfindungsgemäße Kombination werden sämtliche bekannten biochemischen Ursachen des Herzinfarkts und des Reinfarkts prophylaktisch bekämpft, wobei in überraschender Weise festgestellt wurde, daß die bekannt schlechte Magen- und Darmverträglichkeit der Acetylsalicylsäure durch die systemischen Magnesiumwirkungen beseitigt wird. Zusätzlich werden systemische Unverträglichkeiten der Acetylsalicylsäure, wie allergische Überreaktionen, die sich in Verengungen der glatten Muskulatur äußern, durch das Magnesium gehemmt.

Die Magnesiumaminodicarbonsäurehalogenide, die erfindungsgemäß eingesetzt werden, bestehen vorzugsweise aus Magnesium-L-Aspartathydrochlorid oder Magnesium-L-Glutamathydrochlorid. Die Magnesiumaminodicarbonsäurehalogenide, ihre Herstellung und ihre Struktur werden in der Literatur ausführlich beschrieben (vgl. beispielsweise die DE-PS 2228101, die DE-OS 3238118, "Angewandte Chemie", 98. Jhrg., 1986, Heft 11, Seiten 1014 bis 1016 und die DE-PS 1809119).

Als basische Magnesiumaminodicarbonsäuresalze werden vorzugsweise basisches Magnesiummonoaspartat und basisches Magnesiumglutamat verwendet Basisches Magnesiummonoaspartat (Mg(CO$_2$CH$_2$CHNH$_2$CO$_2$) × 3H$_2$O) ist ein weißes Pulver, das bis 300°C nicht schmilzt, und kann in der Weise hergestellt werden, daß 225 g Magnesium-L-diaspartat × 4 H$_2$O in 250 ml H$_2$O dest. angelöst werden und dann eine Kaliumhydroxidlösung (80 g KOH-Plätzchen 85%ig in 100 ml H$_2$O dest.) langsam tropfenweise zugegeben wird bis ein pH-Wert von 10,2-10,6 erreicht ist. Die klare Lösung wird 1 Stunde gerührt. Anschließend wird im Wasserstrahlvakuum (ca. 14 Torr) die Lösung bei 40°C auf 250 bis 275 ml eingeengt und bei Raumtemperatur stehengelassen. Nach ca. 4 Tagen kristallisiert die Lösung durch. Die Kristalle werden über eine Fritte im Wasserstrahlvakuum abgezogen und erst mit wenig H$_2$O destilliert und dann mit Aceton gewaschen. Anschließend werden die Kristalle bis zum Verschwinden des Aceton-Geruchs bei ca. 80-90°C getrocknet.

In ähnlicher Weise läßt sich basisches Magnesiumglutamat herstellen.

Diese basischen Magnesiumamindicarbonsäuresalze werden vorzugsweise dann in den erfindungsgemäßen Mitteln eingesetzt, wenn der Magen der Patienten, denen diese Mittel verabreicht werden, stark übersäuert ist.

Als Magnesiumhalogenid wird vorzugsweise Magnesiumchlorid verwendet.

In zweckmäßiger Weise beträgt in den erfindungsgemäßen Mitteln das Gewichtsverhältnis der Magnesiumverbindung(en) zu der Acetylsalicylsäure 1 : 1 bis 15 : 1.

Die erfindungsgemäßen Mittel werden in besonders bevorzugter Form oral verabreicht, beispielsweise in Form von Kapseln, Tabletten, Granulaten, Pulvern, Brausetabletten und Lösungen, wobei die Einzeldosen (Tagesdosen) der Magnesiumverbindungen 20 bis 240 mg, berechnet als Mg$^{2+}$, und der Acetylsalicylsäure 20 bis 1000 mg betragen können.

Die erfindungsgemäßen Mittel lassen sich in üblicher Weise durch Vermischen der Komponenten und Konfektionieren zu der gewünschten Darreichungsform herstellen, wobei übliche Trägerstoffe, Vedünnungsmittel und/oder Hilfsstoffe eingesetzt werden können.

## Patentansprüche

1. Mittel zur Prophylaxe des Herzinfarkts und zur Verhütung des Reinfarkts, dadurch gekennzeichnet, daß es aus einer Kombination aus Magnesiumaminodicarbonsäurehalogeniden, basischen Magnesiumaminodicarbonsäuresalzen und Magnesiumhalogeniden einzeln oder in Kombination einerseits und Acetylsalicylsäure andererseits sowie gegebenenfalls üblichen pharmazeutischen Trägern, Verdünnungsmittel, und/oder Hilfsstoffen besteht.

2. Mittel nach Anspruch 1, dadurch gekennzeichnet, daß die Magnesiumaminodicarbonsäurehalogenide aus Magnesium-L-Aspartat-Hydrochlorid oder Magnesium-L-Glutamat-Hydrochlorid bestehen.

3. Mittel nach Anspruch 1, dadurch gekennzeichnet, daß die basischen Aminodicarbonsäuresalze aus basischem Magnesiummonoaspartat oder basischem Magnesiummonoglutamat bestehen.

4. Mittel nach Anspruch 1, dadurch gekennzeichnet, daß das Magnesiumhalogenid aus Magnesiumchlorid besteht.

5. Mittel nach den Ansprüchen 1 bis 4, dadurch gekennzeichnet, daß das Gewichtsverhältnis der Magnesiumverbindung (en) zu der Acetylsalicylsäure 1 : 1 bis 15 : 1 beträgt.

6. Mittel nach den Ansprüchen 1 bis 5, dadurch gekennzeichnet, daß es in Form von oral verabreichbaren Formulierungen, Kapseln, Tabletten, Granulaten, Pulvern, Brausetabletten und Lösungen, konfektioniert ist.

## Claims

1. Composition for the prophylaxis of coronary infarct and for the prevention of reinfarct, characterised in that it consists of a combination of magnesium aminodicarboxylate halides, basic magnesium aminodicarboxylate salts and magnesium halides individually or in combination on the one hand and acetylsalicylic acid on the other hand and also, if appropriate, customary pharmaceutical vehicles, diluents, and/or auxiliaries.

2. Composition according to Claim 1, characterised in that the magnesium aminodicarboxylate halides consist of magnesium L-aspartate hydrochloride or magnesium L-glutamate hydrochloride.

3. Composition according to Claim 1, characterised in that the basic aminodicarboxylate salts consist of basic magnesium monoaspartate or basic magnesium monoglutamate.

4. Composition according to Claim 1, characterised in that the magnesium halide consists of magnesium chloride.

5. Composition according to Claims 1 to 4, characterised in that the weight ratio of the magnesium compound(s) to the acetylsalicylic acid is 1 : 1 to 15 : 1.

6. Composition according to Claims 1 to 5, characterised in that it is formulated in the form of orally administrable formulations, capsules, tablets, granules, powders, effervescent tablets and solutions.

## Revendications

1. Agent destiné à la prophylaxie de l'infarctus du myocarde et à la prévention de nouvel infarctus, caractérisé en ce qu'il est constitué d'une combinaison d'halogénhydrates d'aminodicarboxylates de magnésium, de sels basiques de magnésium d'acides aminodicarboxyliques et d'halogénures de magnésium, seuls ou en combinaison d'une part, et d'acide acétylsalicy-lique d'autre part, et éventuellement d'excipients pharmaceu-tiques, diluants et/ou adjuvants usuels.

2. Agent selon l'invention, caractérisé en ce que les halogénhydrates d'aminodicarboxylates de magnésium sont constitués du chlorhydrate du L-aspartate de magnésium ou du chlorhydrate du L-glutamate de magnésium.

3. Agent selon la revendication 1, caractérisé en ce que les sels basiques d'acides aminodicarboxyliques sont constitués de monoaspartate basique de magnésium ou de monoglutamate basique de magnésium.

4. Agent selon la revendication 1, caractérisé en ce que l'halogénure de magnésium est constitué de chlorure de magnésium.

5. Agent selon les revendications 1 à 4, caractérisé en ce que la proportion pondérale du ou des composés du magnésium à l'acide acétylsalicylique est de 1 : 1 à 15 : 1.

6. Agent selon les revendications 1 à 5, caractérisé en ce qu'il est traité pour prendre la forme de formulations administrables par voie orale, de gélules, de comprimés, de granulés, de poudre, de comprimés effervescents et de solutés.